Europäisches Patentamt

European Patent Office

Office européen des brevets

(19)

(11) Veröffentlichungsnummer: **0 226 187**
**A2**

(12) **EUROPÄISCHE PATENTANMELDUNG**

(21) Anmeldenummer: 86117298.9

(22) Anmeldetag: 11.12.86

(51) Int. Cl.⁴: **A61K 33/16** ,
//(A61K33/16,31:19,31:05)

(30) Priorität: 12.12.85 DE 3543801

(43) Veröffentlichungstag der Anmeldung:
24.06.87 Patentblatt 87/26

(84) Benannte Vertragsstaaten:
ES GR

(71) Anmelder: Marquardt, Bernd Dr.
Auf der Heide 1c
D-4040 Neuss(DE)

(72) Erfinder: Marquardt, Bernd Dr.
Auf der Heide 1c
D-4040 Neuss(DE)

(74) Vertreter: Redies, Bernd, Dr. rer. nat.
COHAUSZ & FLORACK Patentanwaltsbüro
Schumannstrasse 97 Postfach 14 01 47
D-4000 Düsseldorf 1(DE)

(54) **Pharmazeutisches Produkt enthaltend ein Gemisch aus Benzoesäure, Phenol und ein Alkalifluorid zur topischen Behandlung von Herpes-Virus-Effluoreszenzen und Verfahren zur Behandlung von Herpes-simplex-Effluoreszenzen am Menschen.**

(57) Die vorliegende Erfindung betrifft pharmazeutische Produkte enthaltend Gemische aus Benzoesäure, Phenol und einem Alkalifluorid in wässriger oder wässrig-alkoholischer Lösung, gegebenenfalls in Anwesenheit anderer üblicher Zusatzstoffe zu pharmazeutischen Zubereitungen in flüssiger oder halbflüssiger Form zur Bekämpfung von durch Herpes-Virus bedingten Erkrankungen am Menschen und Verfahren zur topischen Behandlung von Herpes-simplex-Effluoreszenzen am Menschen mit diesen Produkten.

EP 0 226 187 A2

### Pharmazeutisches Produkt enthaltend ein Gemisch aus Benzoesäure, Phenol und ein Alkalifluorid zur topischen Behandlung von Herpes-Virus-Effluoreszenzen und Verfahren zur Behandlung von Herpes-simplex-Effluoreszenzen am Menschen

Die vorliegende Erfindung betrifft ein neuartiges pharmazeutisches Produkt enthaltend ein Gemisch aus Benzoesäure, Phenol und einem Alkalifluorid in wässriger oder wässrig-alkoholischer Lösung, gegebenenfalls in Anwesenheit anderer üblicher Zusatzstoffe zu pharmazeutischen Zubereitungen in flüssiger oder halbflüssiger Form, und seine Verwendung zur Bekämpfung von durch Herpes-Virus bedingten Erkrankungen am Menschen.

Durch Herpes-Virus bedingte Erkrankungen des Menschen bewirken im allgemeinen mehr oder weniger starke Blasenbildung, insbesondere auf Schleimhäuten beim Menschen die Bildung von zum Teil sehr schmerzenden oder stark juckenden Blasen. Eine besonders bekannte Form derartiger Erkrankungen ist Herpes simplex.

Ein Produkt, das sowohl Benzoesäure als auch Phenol und Natriumfluorid enthält, ist auf dem Markt und wird in stark mit Wasser verdünntem Zustand als Desinfektionsmittel für den Mundraum eingesetzt. Überraschenderweise sind Gemische aus Benzoesäure, Phenol und einem Alkalifluorid, vorzugsweise Natriumfluorid, in wässriger oder wässrig-alkoholischer Lösung, die gegebenenfalls andere übliche Zusatzstoffe zu pharmazeutischen Zubereitungen in flüssiger oder halbflüssiger Form enthalten, in nicht stark mit Wasser verdünnter Form zu einer raschen und wirkungsvollen Bekämpfung von durch Herpes-Virus bedingten Erkrankungen am Menschen, insbesondere zur äußerlichen Behandlung der erkrankten Hautpartien, insbesondere Schleimhautpartien geeignet.

Die einzelnen Bestandteile sind in zahlreichen pharmazeutischen Zubereitungen bekannt. So werden Benzoesäure und Phenol als Antiseptikum eingesetzt und Natriumfluorid wird zur Kariesprophylaxe eingesetzt.

Das erfindungsgemäße pharmazeutische Produkt zur Bekämpfung von durch Herpes-Viren bedingte Erkrankungen am Menschen ist dadurch gekennzeichnet, daß es neben Wasser oder Wasser-Alkohol als Lösungsmittel und neben üblichen Zusatzstoffen zu pharmazeutischen Zubereitungen in flüssiger Form oder halbflüssiger Form auf 100 g Endprodukt 0,1 bis 0,5 g des Alkalifluorids, insbesondere Natriumfluorid, enthält, bevorzugt 0,2 bis 0,4 g Benzoesäure, 0,6 bis 0,8 g Phenol und 0,2 bis 0,4 g des Alkalifluorids.

Beispiel 1

100 g einer wässrigen Lösung enthalten 0,65 g einer 1:5-Lösung von Benzoesäure in Äthylalkohol, 0,73 g Phenol und 0,22 g Natriumfluorid. Das Produkt enthält zusätzlich 5,88 g Mentholöl, 1,2 g 01 Carbophylli, 1,63 g Salizylsäuremethylester und 0,74 g p-Hydroxybenzoesäureäthylester.

Die Bestandteile werden innig vermischt und in Behälter mit einem solchen Verschluß gegeben, mittels dessen des Produkt tropfenweise auf die befallenen Stellen aufgetupft werden kann.

Beispiel 2

100 g einer wässrigen Lösung enthalten 2,4 g einer 1:5-Lösung von Benzoesäure in Äthylalkohol (0,4 g Benzoesäure und 2,0 g 96%-igem Alkohol), 1 g Phenol und 0,4 g Natriumfluorid. Sie enthält keine Zuzatzstoffe z.B. zur Verbesserung des Geruchs. Die Bestandteile werden wie im Beispiel 1 verarbeitet.

**Ansprüche**

1. Pharmazeutisches Produkt enthaltend ein Gemisch aus Benzoesäure, Phenol und einem Alkalifluorid, in wässriger oder wässrig-alkoholischer Lösung, gegebenenfalls in Anwesenheit anderer üblicher Zusatzstoffe zu pharmazeutischen Zubereitungen in flüssiger oder halbflüssiger Form zur topischen Behandlung von Herpes-Virus-Effluoreszenzen.

2. Pharmazeutisches Produkt gemäß Anspruch 1, dadurch gekennzeichnet, daß das Alkalifluorid Natriumfluorid ist.

3. Pharmazeutisches Produkt gemäß Anspruch 1 oder 2, dadurch gekennzeichnet, daß das Produkt auf 100 g 0,1 bis 0,5 g Benzoesäure, 0,5 bis 1,0 g Phenol und 0,1 bis 0,5 g des Alkalifluorids enthält.

4. Pharmazeutisches Produkt gemäß Anspruch 3, dadurch gekennzeichnet, daß es auf 100 g 0,2 bis 0,4 g Benzoesäure, 0,6 bis 0,8 g Phenol und 0,2 bis 0,4 g des Alkalifluorids enthält.

5. Verfahren zur topsichen Behandlung von Herpes-simplex-Effluoreszenzen, dadurch gekennzeichnet, daß ein Produkt der Ansprüche 2 bis 4 auf die Effluoreszenz ein-oder mehrmals aufgebracht wird.